# EUROPEAN PATENT SPECIFICATION

(11) **EP 0 797 969 B1**
(45) Date of publication and mention of the grant of the patent: **08.08.2001**
(21) Application number: 97301953.2
(22) Date of filing: 21.03.1997
(51) Int. Cl.: A61F 13/15

(54) **Disposable diaper**
Wegwerfwindel
Couche jetable

(30) Priority: 28.03.1996 JP 7486496; 28.03.1996 JP 7486596
(43) Date of publication of application: 01.10.1997
(73) Proprietor: UNI-CHARM CORPORATION, Kawanoe-shi Ehime-ken (JP)
(72) Inventor: Kitaoka, Hideaki, Funabashi-shi, Chiba-ken (JP); Hisada, Kenichi, Kawanoe-shi, Ehime-ken (JP); Igaue, Takamitsu, Kawanoe-shi, Ehime-ken (JP); Sasaki, Toru, Kawanoe-shi, Ehime-ken (JP)
(74) Representative: Parry, Christopher Stephen

(56) References cited:
- EP-A- 0 172 036
- EP-A- 0 321 732
- EP-A- 0 350 442
- WO-A-91/00720
- US-A- 3 779 246

## Description

The present invention relates generally to a disposable diaper and, more particularly, to a disposable diaper for infants and adults for absorbing and containing urine and other body exudates.

Conventionally, a plastic film such as polyethylene has been employed as a basic material for a liquid-impermeable backsheet of disposable diapers. A disposable diaper employing a composite sheet of such plastic film and a fibrous nonwoven fabric bonded to an outer surface of the plastic film as the backsheet is known, for example, in Japanese Patent Application Laid-Open No Hei 3-86159.

EP-A-350442 discloses a disposable diaper comprising a liquid permeable topsheet and a backsheet composed of an impermeable plastic layer and an outer protective fibrous layer. A liquid absorbent core is disposed between the topsheet and the backsheet. Side flaps formed by portions of the topsheet and the backsheet extend outwardly beyond transversely opposite side edges of the core and elastic members are secured along leg-surrounding curved rows formed on transversely opposite sides of the diaper. The plastic layer, however, is only a relatively narrow strip in the central area of the diaper and is significantly smaller than the absorbent core and covers only a small part of the outer fibrous layer.

The diapers employing the backsheet of plastic film often cannot get into consumers' favour due to a touch peculiar to the plastic film. According to the diaper employing the foregoing composite sheet as the backsheet, on the other hand, an outer surface of such plastic film is covered with a nonwoven fabric which provides an outer surface of the backsheet with a cloth-like touch and thereby eliminates a problem raised by such plastic film. However, the backsheet comprising composite sheet necessarily has a rigidity higher than that exhibited by the backsheet comprising a plastic film alone and gathers will be formed along a waist-opening and leg-openings as elastic members provided along these openings contract at a pitch larger than in the case of the backsheet comprising a plastic film alone. Particularly when gathers are formed around the wearer's legs at a relatively large pitch, in other words, when it is impossible to obtain fine gathers around the wearer's legs, a desired fitness of the diaper around the wearer's legs can not be expected and, in consequence, leakage of body exudates cannot be avoided.

In view of the above-mentioned problems, it is a principal object of the invention to improve a touch of front and rear waist region as well as a fitness around the wearer's legs in a disposable diaper.

The object set forth above is achieved, according to the invention, by a disposable diaper comprising a liquid-permeable topsheet, a liquid-impermeable backsheet composed of a laminate of a plastic film layer and a fibrous nonwoven fabric layer integrally bonded to an outer surface of the plastic film, a liquid-absorbent core disposed between the topsheet and the backsheet, side flaps formed by portions of the topsheet and the backsheet extending outwardly beyond transversely opposite side edges of the core, and elastic members secured along leg-surrounding curved zones formed on transversely opposite sides of the diaper; characterised in that the plastic film layer and the fibrous nonwoven fabric layer substantially completely cover one another except in the leg surrounding curved zones in which one or other of these layers is cut away; each of the leg-surrounding curved zones being defined by an inner side edge formed by the outer edge of the cut away layer and a curved outer side edge formed by the outer edge of the other layer such that the rigidity of each of the leg-surrounding curved zones is lower than that of the remaining portions of the side flap of which these zones are part; and in that an inner side edge of the elastic member secured along the leg-surrounding curved zone is spaced outwardly of the inner side edge of the cut away layer by a distance of 5 - 35mm.

In the diaper according to the invention, the elastic member secured in the leg-surrounding curved zone can sufficiently contract to achieve a good fitness of the leg-surrounding curved zone around the wearer's legs, since a rigidity of the leg-surrounding curved zone is lower than that of the side flap except the leg-surrounding zone.

Other and another objects, features and advantages of the invention will appear more fully from the following description.
Figure 1 is a plan view of a disposable diaper as a first embodiment of the invention, as partially broken away;
Figure 2 is a sectional view taken along line II-II in Figure 1;
Figure 3 is a view similar to Figure 1 but showing a variant of the invention;
Figure 4 is a plan view of a disposable diaper as a second embodiment of the invention, as partially broken away; and
Figure 5 is a sectional view taken on line V-V in Figure 4.

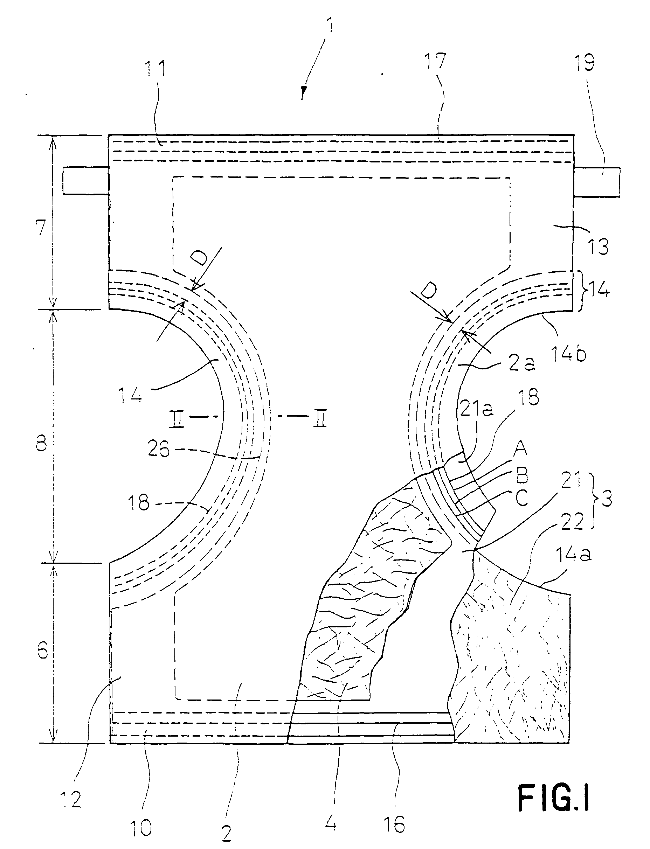

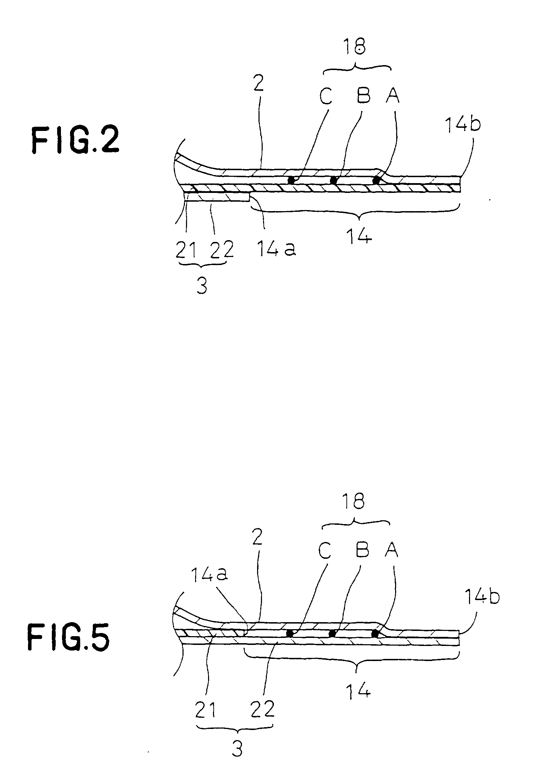

A first embodiment of the invention is illustrated by a disposable diaper 1 of Figure 1 in a plan view as partially broken away and Figure 2 is a sectional view taken along line II-II in Figure 1. The diaper 1 comprises a liquid-permeable topsheet 2, a liquid-impermeable backsheet 3 and a liquid-absorbent core 4 disposed therebetween, and has a front waist region 6, a rear waist region 7 and a crotch zone 8 extending therebetween. The topsheet 2 and the backsheet 3 extend outward beyond a peripheral edge of the core 4 so as to define longitudinally opposite end flaps 10, 11 and transversely opposite side flaps 12, 13 and are bonded together along at least peripheries of the end flaps 10, 11 and side flaps 12, 13. Leg-surrounding curved zones 14 for a good fitness around the wearer's legs are formed on transverse sides of the side flaps 12, 13, with the side flaps 12, 13 curved inwardly thereof. Elastic members 16, 17 each comprising three elastic elements are disposed in their longitudinally extended conditions between the topsheet 2 and the backsheet 3 along the longitudinally opposite end flaps 10, 11 and elastic members 18, 18 each comprising three elastic elements (A, B, C) are also disposed in their longitudinally extended conditions between the topsheet 2 and the backsheet 3 along each leg-surrounding curved region 14. Tape fasteners 19 extend outwardly from transversely opposite side edges 13 of the rear waist region 7 so that these tape fasteners 19 may be detachably fastened to the corresponding transversely opposite side edges 12 of the front waist region 6.

The topsheet 2 is shaped in a sandglass well known to those skilled in the art and may be made of a liquid-permeable nonwoven fabric or a liquid-permeable perforated plastic film.

The backsheet 3 comprises a fluid-impermeable or air-permeable but liquid-impermeable plastic film 21 entirely covering an outer side of the diaper 1 and a fibrous nonwoven fabric 22 integrally bonded to an outer surface of the plastic film 21. The plastic film 21 is substantially the same in shape and in size as the topsheet 2 while the nonwoven fabric 22 is also substantially the same in shape and in size as the plastic film 21 except in the leg-surrounding curved zones 14. The nonwoven fabric 22 is cut off in each of the leg-surrounding curved zones 14 which is defined by a curved inner side edge 14a and a curved outer side edge 14b extending oppositely to the curved inner side edge 14a.

The elastic members 18 are each secured by hot melt adhesive (not shown) between portions 2a, 21a of the top sheet 2 and the plastic film 21 of the backsheet 3 defining each of the leg-surrounding curved zones 14. In each of the leg-surrounding curved zones 14, the curved inner side edge 14a is spaced apart from an inner side edge of the one (C) of three elastic elements (A, B, C) that is proximate to the curved inner side edge 14a by a distance D of 5 - 35mm.

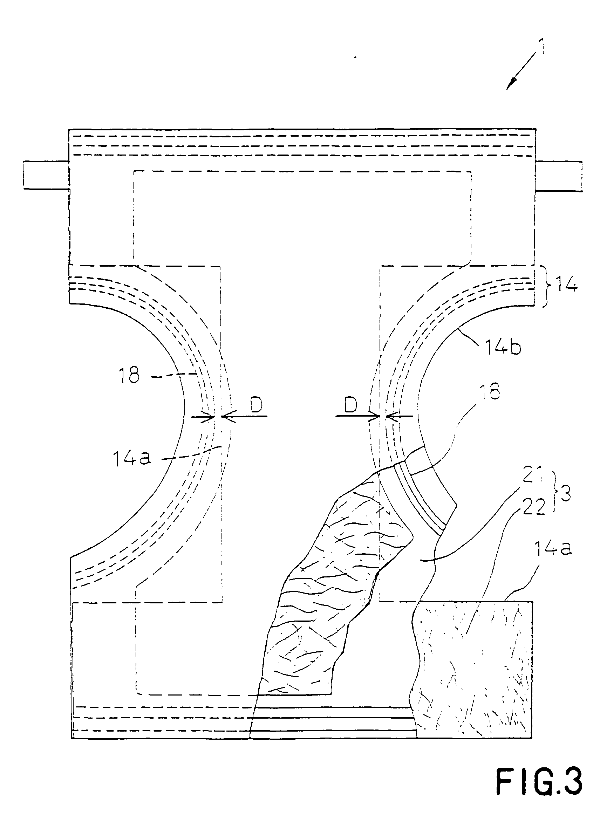

Figure 3 shows a variant of the diaper 1 constructed according to the invention. In this diaper 1, the nonwoven fabric 22 is cut off substantially in a square U-shape in each leg-surrounding curved region 14. The innermost edge of the elastic members 18 each is spaced from an inner side edge 14a of said square U-shaped nonwoven fabric 22 by D of 5 - 35mm.

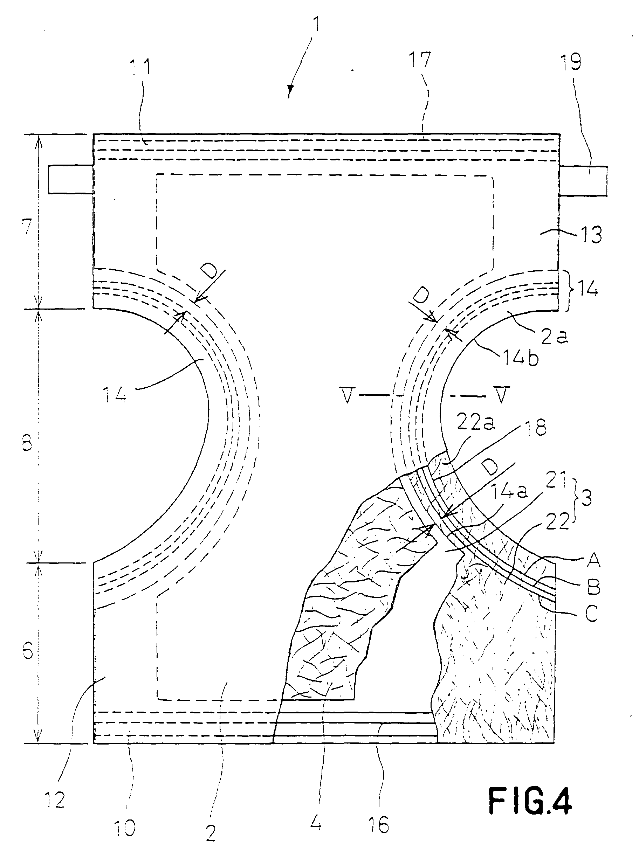

A second embodiment of the invention is illustrated by a disposable diaper 1 of Figure 4 in a plane view as partially broken away and Figure 5 in a sectional view taken along V-V in Figure 4, where components similar to these previously described have the same reference numeral and the description thereon is omitted in this embodiment.

The backsheet 3 comprises a fibrous nonwoven fabric which is substantially the same in shape and in size as the topsheet 2 and a fluid-impermeable, or an air-permeable but liquid-impermeable plastic film integrally bonded to an inner surface of the nonwoven fabric 22. The plastic film 21 is substantially the same in shape and in size as the nonwoven fabric 22 except in the leg-surrounding curved zones 14 which are defined by a curved inner side edge 14a and a curved outer side edge 14b extending oppositely to the curved inside edge 14a. The plastic film 21 is cut off in each of the leg-surrounding curved zones 14. The elastic members 18 are each secured by hot melt adhesive (not shown) between portions 2a, 22a of the topsheet 2 and the nonwoven fabric 22 of the backsheet 3 defining each of the leg-surrounding curved zones 14. In each of the leg-surrounding curved zones 14, the curved inner side edge 14a is spaced apart from the inner side edge of the one (C) of three elastic elements (A, B, C) that is proximate to the curved inner side edge 14a by a distance D of 5 - 35mm.

In order to avoid exudation of body exudates through the portion 22a of the nonwoven fabric 22 in the leg-surrounding curved zone 14, the nonwoven fabric 22 is preferably made of hydrophobic fibre and appropriately treated so as to have a hydraulic pressure resisting value of 20mm or higher as determined according to Japanese Industrial Standards L 1092.

With the diaper 1 constructed as has been described above, the transversely opposite outermost surface of the front and rear waist regions 6, 7 as well as the crotch region 8 with which the hands of the wearer or his or her mother may often come in contact is defined by the nonwoven fabric 22 advantageously presenting a cloth-like touch and the nonwoven fabric 22 is free from exudation of body exudates because the nonwoven fabric 22 is lined with the plastic film 21. It is important to mind that the leg-surrounding curved zones 14 comprise the plastic film 21 alone or the nonwoven fabric 22 alone and the absence of the plastic film 21 or the nonwoven fabric 22 in these zones 14 correspondingly decreases a rigidity of the backsheet 3 in these zones 14. Contraction of the elastic members 18 causes relatively fine gathers to be formed in the leg-surrounding curved zones 14 and such fine gathers contribute to improvement of a fitness of the diaper 1 around the wearer's legs as well as an anti-leakage effect.

The foregoing distance D is preferably 5 - 35mm and this distance D is preferably adjusted to a relatively large for the adult-sized diaper. A distance D less than 5 mm may result in a situation, depending on a correlation between a rigidity of the core and an extensional stress of the elastic member 18 but often with the core and the elastic member commonly exployed in the diaper as herein discussed, that the elastic member 18 cannot sufficiently contract to achieve the desired fitness of the diaper 1 around the wearer's legs.

Bonding of the nonwoven fabric 22 to the plastic film 21 may be achieved using adhesive such as hot melt type adhesive or using a heat-sealing technique as for bonding of the other diaper components.

## Claims

1. A disposable diaper (1) comprising a liquid-permeable topsheet (2), a liquid-impermeable backsheet (3) composed of a laminate of a plastic film layer (21) and a fibrous nonwoven fabric layer (22) integrally bonded to an outer surface of the plastic film, a liquid-absorbent core (4) disposed between the topsheet and the backsheet, side flaps (12,13) formed by portions of the topsheet and the backsheet extending outwardly beyond transversely opposite side edges of the core, and elastic members (16,17) secured along leg-surrounding curved zones (14) formed on transversely opposite sides of the diaper;
characterised in that the plastic film layer (21) and the fibrous nonwoven fabric layer (22) substantially completely cover one another except in the leg surrounding curved zones (14) in which one or other of these layers is cut away; each of the leg-surrounding curved zones (14) being defined by an inner side edge (14a) formed by the outer edge of the cut away layer and a curved outer side edge (14b) formed by the outer edge of the other layer such that the rigidity of each of the leg-surrounding curved zones (14) is lower than that of the remaining portions of the side flap of which these zones are part;
and in that an inner side edge of the elastic member secured along the leg-surrounding curved zone is spaced outwardly of the inner side edge of the cut away layer by a distance of 5 - 35mm.

2. A disposable diaper according to Claim 1,
wherein the nonwoven fabric layer (22) is cut away along the leg-surrounding curved zones (14).

3. A disposable diaper according to Claim 1,
wherein the plastic film layer (21) is cut away along the leg-surrounding curved zone.

4. A disposable diaper according to Claim 3,
wherein the nonwoven fabric layer (22) is made of hydrophobic fibre and has a hydraulic pressure resisting value of at least 20mm.

5. A disposable diaper according to Claim 2,
wherein each of the elastic members is disposed between portions of the topsheet (2) and the plastic film layer (21) of the backsheet in the leg-surrounding curved zone (14).

6. A disposable diaper according to Claim 3,
wherein the elastic members are disposed between portions of the topsheet (2) and the nonwoven fabric layer (22) of the backsheet in the leg-surrounding zones (14).

## Patentansprüche

1. Wegwerfwindel (1) bestehend aus einer flüssigkeitsdurchlässigen Deckschicht (2), einer flüssigkeitsundurchlässigen Rückschicht (3), die aus einem Laminat einer Plastfolienlage (21) und einer fibrösen Faservlieslage (22) zusammengesetzt ist, die integral an eine Außenfläche der Plastfolie geklebt ist, einer flüssigkeitsabsorbierenden Kernschicht (4), die zwischen der Deckschicht und der Rückschicht angeordnet ist, seitlichen Klappen(12, 13), die durch Teile der Deckschicht und der Rückschicht gebildet sind, die sich nach außen über quergerichtete, entgegengesetzte Seitenränder der Kernschicht erstrecken, und elastischen Elementen (16,17), die entlang das Bein umgebender bogenförmiger Zonen (14) befestigt sind, die an quergerichteten, entgegengesetzten Seiten der Wegwerfwindel geformt sind;
dadurch gekennzeichnet, dass die Plastfolienlage (21) und die fibröse Faservlieslage (22) einander im wesentlichen, mit Ausnahme in den das Bein umgebenden bogenförmigen Zonen (14), in denen die eine oder andere dieser Lagen weggeschnitten ist, völlig decken; jede der das Bein umgebenden bogenförmigen Zonen (14) wird durch einen inneren Seitenrand (14a), der durch den Außenrand der weggeschnittenen Lage geformt ist, und einen bogenförmigen äußeren Seitenrand (14b), der durch den Außenrand der anderen Lage so geformt ist, dass die Steifigkeit jeder der das Bein umgebenden Zonen (14) geringer als jene der übrigen Teile der Seitenklappe ist, die Teil dieser Zonen sind, definiert;
und, dadurch gekennzeichnet, dass ein innerer Seitenrand des elastischen Elements, das entlang der das Bein umgebenden Zone befestigt ist, vom inneren Seitenrand der weggeschnittenen Lage nach außen einen Abstand von 5 - 35 mm aufweist.

2. Wegwerfwindel nach Anspruch 1, worin die Faservlieslage (22) entlang der das Bein umgebenden Zonen (14) weggeschnitten ist.

3. Wegwerfwindel nach Anspruch 1, worin die Plastfolienlage (21) entlang der das Bein umgebenden Zone weggeschnitten ist.

4. Wegwerfwindel nach Anspruch 3, worin die Faservlieslage (22) aus wasserabweisender Faser hergestellt ist und einen Wasserdruck-Widerstandswert von mindestens 20 mm aufweist.

5. Wegwerfwindel nach Anspruch 2, worin jedes der elastischen Elemente zwischen Teilen der Deckschicht (2) und der Plastfolienlage (21) der Rückschicht in der das Bein umgebenden Zone (14) angeordnet ist.

6. Wegwerfwindel nach Anspruch 3, worin die elastischen Elemente zwischen Teilen der Deckschicht (2) und der Faservlieslage (22) der Rückschicht in den das Bein umgebenden Zonen (14) angeordnet sind.

## Revendications

1. Couche jetable (1) comprenant une feuille supérieure (2) perméable aux liquides, une feuille arrière (3) imperméable aux liquides composée d'un stratifié formé d'une épaisseur (21) de film plastique et d'une épaisseur (22) d'étoffe non tissée fibreuse attachée d'un seul tenant à une surface extérieure du film plastique, un noyau absorbeur (4) de liquides disposé entre la feuille supérieure et la feuille arrière, des rabats latéraux (12, 13) formés par des parties de la feuille supérieure et de la feuille arrière qui s'étendent vers l'extérieur au-delà de bords latéraux du noyau transversalement opposés, et des organes élastiques (16, 17) fixés le long de zones incurvées (14) qui entourent les jambes et sont formées sur des côtés transversalement opposés de la couche;
caractérisée en ce que l'épaisseur (21) de film plastique et l'épaisseur (22) d'étoffe non tissée fibreuse se recouvrent sensiblement complètement l'une l'autre, sauf dans les zones incurvées (14) entourant les jambes dans lesquelles l'une ou l'autre de ces épaisseurs est enlevée par découpe, chacune des zones incurvées (14) entourant les jambes étant définie par un bord latéral intérieur (14a) formé par le bord extérieur de l'épaisseur découpée, et par un bord latéral extérieur incurvé (14b) formé par le bord extérieur de l'autre épaisseur de façon que la rigidité de chacune des zones incurvées (14) entourant les jambes soit plus faible que celle des parties restantes du rabat latéral dont ces zones font partie;
et en ce que le bord latéral intérieur de l'organe élastique fixé le long de chaque zone incurvée entourant les jambes est espacé vers l'extérieur, d'une distance de 5 à 35 mm, du bord latéral intérieur de l'épaisseur enlevée par découpe.

2. Couche jetable selon la revendication 1, dans laquelle l'épaisseur (22) d'étoffe non tissée est enlevée par découpe le long des zones incurvées (14) entourant les jambes.

3. Couche jetable selon la revendication 1, dans laquelle l'épaisseur (21) de film plastique est enlevée par découpe le long des zones incurvées entourant les jambes.

4. Couche jetable selon la revendication 3, dans laquelle l'épaisseur (22) d'étoffe non tissée consiste en une fibre hydrophobe dont la valeur de résistance à la pression hydraulique est au moins de 20 mm.

5. Couche jetable selon la revendication 2, dans laquelle chacun des organes élastiques est disposé, dans la zone incurvée (14) entourant les jambes, entre des parties de la feuille supérieure (2) et de l'épaisseur (21) de film plastique de la feuille arrière.

6. Couche jetable selon la revendication 3, dans laquelle les organes élastiques sont disposés, dans les zones (14) entourant les jambes, entre des parties de la feuille supérieure (2) et de l'épaisseur (22) d'étoffe non tissée de la feuille arrière.
